# EUROPEAN PATENT APPLICATION

(11) **EP 1 621 639 A2**
(43) Date of publication of application: **01.02.2006**
(21) Application number: 05019760.7
(22) Date of filing: 12.05.1999
(51) Int. Cl.: C12Q 1/68, G01N 33/53, G01N 33/574, G01N 33/567

(54) **A novel method of diagnosing, monitoring and staging prostate cancer**

(30) Priority: 21.05.1998 US 86265 P
(62) Divisional of application: 99921946.2
(71) Applicant: Diadexus, Inc., South San Francisco, CA 94080 (US)
(72) Inventor: Ali, Shujath, Santa Clara CA 95051 (US); Salceda, Susana, San Jose CA 95136 (US); Sun, Yongming, San Jose CA 95128 (US); Cafferkey, Robert, Mountain View CA 94041 (US)
(74) Representative: Peebles, Katrina

(57) **Abstract**

The present invention provides a new method for detecting, diagnosing, monitoring, staging and prognosticating prostate cancer.

SEQUENCE LISTING

<110> Ali, Shujath
      Salceda, Susana
      Sun, Yangming
      Cafferkey, Robert

<120> A Novel Method of Diagnosing, Monitoring and Staging
      Prostate Cancer

<130> DEX-0034

<140>
<141>

<150> 60/086,265
<151> 1998-05-21

<160> 7

<170> PatentIn Ver. 2.0

<210> 1
<211> 1936
<212> DNA
<213> Homo sapiens

<220>
<221> unsure
<222> (1908)

<400> 1
aatggtatgc caacttaagt atttacaggg tggcccaaat agaacaagat gcactcgctg 60
tgattttaag acaagctgta taaacagaac tccactgcaa gagggngggc cgggccagga 120
gaatctccgc ttgtccaaga caggggccta aggagggtct ccacactgct gctaggggct 180
gttgcatttt tttattagta gaaagtggaa aggcctcttc tcaacttttt tcccttgggc 240
tggagaattt agaatcagaa gtttcctgga gttttcaggc tatcatatat actgtatcct 300
gaaaggcaac ataattcttc cttccctcct tttaaaattt tgtgttcctt tttgcagcaa 360
ttactcacta aagggcttca ttttagtcca gatttttagt ctggctgcac ctaacttatg 420
cctcgcttat ttagcccgag atctggtctt ttttntgtnt tttttttntt tccgtctccc 480
caaagcttta tctgtcttga ctttttaaaa aagtttgggg gcagattctg aattgggcta 540
aaagacatgc atttttaaaa ctaggcaact tcttatttct ttcctttaaa aatacatagc 600
attaaatccc aaatcctatt taaagacctg acagcttgag aaggtcacta ctgcatttat 660
aggaccttct ggtggttctg ctgttacgtt tgaagtctga caatccttga gaatctttgc 720
atgcagagga ggtaagaggt attggatttt cacagaggaa gaacacagcg cagaatgaag 780
ggccaggctt actgaggctg tccagtggag ggctcatggg tgggacatgg aaaagaaggc 840
agcctaggcc ctggggagcc cagtccactg agcaagcaag ggactgagtg agccttttgc 900
aggaaaaggc taagaaaaag gaaaaccatt ctaaaacaca acaagaaact gtccaaatgc 960

tttgggaact gtgtttattg cctataatgg gtccccaaaa tgggtaacct agacttcaga 1020
gagaatgagc agagagcaaa ggagaaatct ggctgtcctt ccattttcat tctgttatct 1080
caggtgagct ggtagagggg agacattaga aaaaaatgaa acaacaaaac aattactaat 1140
gaggtacgct gaggcctggg agtctcttga ctccactact taattccgtt tagtgagaaa 1200
cctttcaatt ttcttttatt agaagggcca gcttactgtt ggtggcaaaa ttgccaacat 1260
aagttaatag aaagttggcc aatttcaccc cattttctgt ggtttgggct ccacattgca 1320
atgttcaatg ccacgtgctg ctgacaccga ccggagtact agccagcaca aaaggcaggg 1380
tagcctgaat tgctttctgc tctttacatt tcttttaaaa taagcattta gtgctcagtc 1440
cctactgagt actctttctc tcccctcctc tgaatttaat tctttcaact tgcaatttgc 1500
aaggattaca catttcactg tgatgtatat tgtgttgcag ngaaaagaaa aaagtgtctt 1560
tgtttaaaat tacttggttt gtgaatccat cttgcttttt ccccattgga actagtcatt 1620
aacccatctc tgaactggta gaaaaacatc tgaagagcta gtctatcagc atctgacagg 1680
tgaattggat ggttctcaga accatttcac ccagacagcc tgtttctatc ctgtttaata 1740
aattagtttg ggttctctac atgcataaca aaccctgctc caatctgtca cataaaagtc 1800
tgtgacttga agtttagtca gcacccccac caaactttat ttttctatgt gttttttgca 1860
acatatgagt gttttgaaaa taaagtaccc atgtctttat taaaaaanaa aaaaaagggc 1920
ggccgccgac tagtga 1936

<210> 2
<211> 637
<212> DNA
<213> Homo sapiens

<400> 2
gtaggggcag acttactgcc ttgaacgaaa gacgatggtc ctcgctcagc ctcactccaa 60
ttatgttcct ctaggtgggg caggtagggg gtccagcttc ctgcttgctg gtggttcagg 120
tcatgcgtcc agccttgtcc cttctgacct gggccctacc cacggggaaa tgttcccata 180
gcagaagaat cagccccaca gtgcaggggt gtgttagtgg ggaacgggct ctgggctcct 240
gtgggaacca gggaccccct atcttggtac cggtcattgg atgtatcccc agctcatgcc 300
tgtgtctgtc ttggcccgtg tggtcaccct gtgttcatct ctctcccagc catggcctct 360
caaactgggg ttttcgtctc cctatgaggg ggtcctggta tgtacgcgtt cggtgggccc 420
gcggtgcatg tctcccggtg cagtgcatgc tggggttccc tggggccctg ggcccctcgt 480
aggatagaca gagcctgtcc taaccttccg gaagtgcatg ctggggaggc cccttgcctg 540
ctgaccttct gtgctcagga cgactaatcg gccacatgac caccactctg tcccatggga 600
ttcctagaga agtctcacta agagcccagc acactca 637

<210> 3
<211> 2693
<212> DNA
<213> Homo sapiens

<220>
<221> unsure
<222> (2266)..(2512)

<220>
<221> unsure
<222> (586)

<220>
<221> unsure
<222> (1480)

<220>
<221> unsure
<222> (1532)

<220>
<221> unsure
<222> (1562)..(1566)

<220>
<221> unsure
<222> (1569)

<220>
<221> unsure
<222> (1571)

<220>
<221> unsure
<222> (1631)

<400> 3
gctcctacag ccgcatctgc gttaacatag catccctatg gccactgtct cccttgatcc 60
ccacagccat cctaggagaa aggcagaatg tcataatttg ctaaaaggga tgctgaggct 120
ctgggaggga aagggacttg cctaaagccc cagggtgaag cagcatctct ggactcccag 180
tccagtgatc ttgcccaata ctttgctgct tgcctatacc cctctaactt ggtcaacagc 240
acatcacagg gcaagcccaa tccctgcttc attttatat atgggcgctg gtccacagcc 300
ccactctcca gccatttgga aacaaaaaca gatgctattg ttcttcctta gagaacgtgg 360
ccagtggaga cggcacactg gaaatcagag tgaatgttct tgaaagaggg tcacgggtca 420
acaaggccca gccaaaggat gcagtagaac cattttcctt agaaatcttt gggagtgaag 480
taggcttcag ccactaccca tccctgccct tgcggctacc actaccccat tagtttagac 540
agggtcgggc ggggaggggt gtggagaaga aatgagcttg cctgtngccc ccaggctccc 600
tctgtcctag ctcaggtctg ggtgccattc tttacactcg tgtgctcgct cacgcacaca 660
tcacacacct tgctggtcac acagtcacag actcgcctct gctcctgtgg tccagtggcc 720
ggacaccccc tgggatggct caaaggagtc aggacttgga agtggggaca tcagggtagc 780
tgaaggaaat ccacacaccc agagcatctc ggagttcaga ctctcagacc tgaagtaggc 840
gcccccggga ctgggctagg agttggacgg aatggaggat ggaggacagc gagaagaaag 900
gaagagaaat gcaaagtgtg ggcagccgcc aagagtgaaa atagagggaa gtgtcatgca 960
agtgctggac agaaggcggc aggtgggacg agccccacag ccccctcctc aaaaacgacc 1020
acctccagga ctcagtgatc cctgggggc aggctctgcc agccctcggc cacacgtggc 1080
tccggcaccc atggtcccag tgccttggat ggagacggcc agttctggcg gccagatgtg 1140
gtgctctgga atccagtccc atttccttcc tggccacgcc tgttccagcg gcctctttgg 1200
ctgcattcag cccctactta cctggggacc ccggctgggg cacaagagca ccaggggggt 1260
agggcccaaa gggatcaggg gaagcctctg gcctggaggg tatggggcac gcttccccaa 1320

gggcggaccc ggcaggagga agcccaggag ctgggtcctg ccgcccagga gctgggccct 1380
gccacccagg ccgggctagg gacatggcag ggcctgggca tcctgacgct ggacttgggc 1440
gacctgggag gcacagggag gggagagatg ggcggccccn acccagcgca gtgccggcca 1500
caccccaagg cggttgccag agcttaaggc cnggccccag caggagaaca tcccagctcc 1560
annnnnccnc nccgcagcca gtgctccttg tcaagctccc cccgtcactc caggtgggag 1620
ccaccccggt naggggggtgt gccacttgcc cccagggcac tcctctgggc atcccgggtg 1680
ggggattttg gggccgtggg gggcagtctc tggtacctgt gtgcgtcagg gatgctctgc 1740
acctgcaacc aggtgtcgtc cacgggcggg ggcatgggca tggtgacagt ggtcctgttg 1800
atgtcaccga tgatgctgag cgcctccttc agcgcgtggt gcatgtgcag catctcgtcg 1860
tgctgctgtg cctgctctgc caactcctcc atcagtgtgt tctggttccc acatgagtac 1920
atattggcca gcggctccga gatgatgaac tccggggtct gagagtgggc aaacagggaa 1980
gaaggttggg acctggtgcc tgtgccgccc tggctgcctt gctgggccct tctgggactg 2040
tgcgctggac ttggagcccc ttggagtatg gcttttcaca cgggcttcta taccgcttcg 2100
actggaagat ccacctcccc actgcctttt ctcactcaga tggggacacc gaggtccaga 2160
ggaaaagaca cctgtcaaat gtcacagatc tgggagggga cttaagacct atcatgccaa 2220
gaggacacct gtctactcag tttttttttg gtggggcggg gggcgnnnnn nnnnnnnnnn 2280
nnnnnnnnnn nnnnnnnnnn nnnnnnnnnn nnnnnnnnnn nnnnnnnnnn nnnnnnnnnn 2340
nnnnnnnnnn nnnnnnnnnn nnnnnnnnnn nnnnnnnnnn nnnnnnnnnn nnnnnnnnnn 2400
nnnnnnnnnn nnnnnnnnnn nnnnnnnnnn nnnnnnnnnn nnnnnnnnnn nnnnnnnnnn 2460
nnnnnnnnnn nnnnnnnnnn nnnnnnnnnn nnnnnnnnnn nnnnnnnnnn nnggagttgg 2520
agttgatgcc tggatacagg agctctgtgg gtgggagtga gacaaaacac agggtcctga 2580
gctctgggga ccaagcaatg tcctctggtg aaaaaaatcc tggacttgct ggcagaagat 2640
ttgcctctta cttgccatgt gctctgaata catttacctg ccctctggga aaa 2693

<210> 4
<211> 292
<212> DNA
<213> Homo sapiens

<220>
<221> unsure
<222> (284)

<400> 4
aagaatatga gatttgctta gaaatgaagg actggaagga gcccacagag ttatttttta 60
aactatccag taaggcttag agggtttcaa tcagaaatat gtgttagggg aaaaaatgca 120
ctttttctat attaaaaaat attattttct tcttttaaat gtaaagcatt cctattgtga 180
agaattgaga aaatacagaa aagtacaaag aaaaacatta cctacaactc caccatccgt 240
gattatcact gttcacattt gtggctcatt tttcagtatk tctnttattt aa 292

<210> 5
<211> 2694
<212> DNA
<213> Homo sapiens

<220>
<221> unsure
<222> (52)

<220>
<221> unsure
<222> (74)

<220>
<221> unsure
<222> (76)

<220>
<221> unsure
<222> (80)

<220>
<221> unsure
<222> (92)

<220>
<221> unsure
<222> (97)

<220>
<221> unsure
<222> (123)

<220>
<221> unsure
<222> (132)

<220>
<221> unsure
<222> (173)

<220>
<221> unsure
<222> (217)

<220>
<221> unsure
<222> (257)

<220>
<221> unsure
<222> (2539)

<400> 5
   tactatattg ctcagcattt ctaagtattc tctaagtgct ctttatttat gntttaaaat 60
   agctctctta cccngntgcg ncgactagaa gancttgntt taggaaacaa tgaaatatat 120
   aanttgccag antcaattgg agccctctta catctaaaag atctctggtt ggntggaaat 180
   caactgtcag aattacctca ggaaatagga aatctgnaga acctgctgtg tttagatgtc 240
   tctgaaaaca ggttggnaag acttcctgaa gaaatcagtg gcctgacttc attaacggat 300
   ttagtcattt cccagaactt attagaaacg attccggatg gcattggaaa actaaagaaa 360
   ctgtcaatct tgaaggtgga tcagaataga ctcacacagt tgcctgaagc agttggggaa 420
   tgtgaaagtc tcactgagtt agttcttaca gaaaatcagc tcctgaccct gcctaaaagc 480
   attggaaaac taaagaagtt gagcaacttg aatgcagaca gaaataaatt agtgtcctta 540
   ccaaaagaga tcggcgggtg ctgcagcctc actgtgttct gtgtacgtga caacagacta 600
   actcggatac ctgcagaggt gtcacaggca acagaacttc atgtcctgga tgtggcaggg 660
   aacaggttgc tgcatctacc tttatccctg actgccttga agttgaaggc tctgtggcta 720
   tctgacaacc agtcccagcc cctgcttaca ttccagacag acacagacta caccacagga 780
   gagaagattt taacctgtgt cttacttcct cagctgcctt ctgaacctac ttgtcaagag 840
   aatctgcctc gctgtggtgc actggagaac ttggtaaatg atgtctctga tgaagcctgg 900
   aacgagcgtg ctgtcaacag agtcagtgcg atccgatttg tggaggatga gaaagatgaa 960
   gaagacaatg agacgagaac acttctaagg cgagccactc cacacccagg ggagttaaag 1020
   cacatgaaaa agacagtgga gaatttacgg aatgacatga atgctgctaa aggactggac 1080
   tcaaacaaaa acgaggtcaa tcatgccatt gaccgagtga ccacttctgt gtagagtttc 1140
   acctccaagt tttacctcct gtgtcttcct ctgctgtcga gacgttcctg tctgcttccc 1200
   gggagcctca cgtgctcctt gtcctaacca gcccccgcgc gccatcttcc cgtggagtgt 1260
   ggggaagctg ctgtctccca ggaagtgcct tactcatccc gcaaccagtc agcgcaccag 1320
   tggtctcccg gtgtgatttt tttttttttt aatttcagtt gtttgtaata agtagaatac 1380
   actactgtaa acatacgacc tttgtttttg tcttatgttg gggtaaagga aagcaggaag 1440
   gggaattttt atcctcctcc cttccgtaaa gtgctgggat attttgaatc ccccaagttc 1500
   ccttggacct actgatgaga gatagtttta tgtatgggga aaaatggata ctttttaaac 1560
   cttttttggc agctcagatg gtgtaaattt taaaattttg tataggtatt tcataacaaa 1620
   aatatgtatt tcttttttgt tattttatct tgaaaacggt acatatttta gtatttgtgc 1680
   agaaaaacaa gtcctaaagt atttgttttt atttgtacca tccacttgtg ccttactgta 1740
   tcctgtgtca tgtccaatca gttgtaaaca atggcatctt tgaacagtgt gatgagaata 1800
   ggaatgtggt gttttaaagc agtgttgcat tttaatcagt aatctacctg gtggatttgt 1860
   ttttaaccaa aaagatgaat tatcaatgat ttgtaattat atcggttgat tttttttgaa 1920
   aagatgaacc aaaggatttg actgctaata ttttattcct tacacttttt ttctgaataa 1980
   gtctctcata atgagtgcag tgtcagactg tgcctactct gatggtatgt gccatttgta 2040
   aaataaaata gagcagaaaa acacaaaaag agaacactgg ttcagacatt cagtgggcaa 2100
   gtaaattatg gactgcaaaa taatgatttt tattcaagaa agctttaaaa gttttatatc 2160
   cagatataca accacaataa agcaaaataa cctactatca aaatagaaat gttgctatct 2220
   ttataagtgc aatttaattt gtaaatagag tttgaatcaa agtatcacaa aatactgctt 2280
   caagatttaa ttttaaatct gctaatttaa gggatattgg gaaaagtttt ggtgtgtttc 2340
   tgttgatttc ttttttgtat gctgtgataa aagagaaatg aaaagtgcca gtcactgtgt 2400
   ggtgtctagg aaaatcatat atattttttt ctccaagaaa taaattcatc ctggacattg 2460
   gccatacagc tttttaaaat tattactttg tatgttcaag tgatagcagg tagccaaatt 2520
   ctttgacagt gtgctctgnt ctgttaaata tctaaattac ccgtcagttg tgagtgacct 2580
   cctgtgggac ttgcattcac atggggcaga gcccagaatt gcctttgact ctggctagta 2640
   attttgggtt gtggctatct ggccaattgg actccttata aacccgtctt caac 2694

<210> 6
<211> 1335
<212> DNA
<213> Homo sapiens

<220>
<221> unsure
<222> (17)

<400> 6
tcatatagta ggaaganaag cacctaggtt tgaggccagg gctggctgct gtcagaacct 60
aggccctccc ctgccttgct ccacacctgg tcaggggaga gaggggagga aagccaaggg 120
aagggaccta actgaaaaca aacaagctgg gagaagcagg aatctgcgct cgggttccgc 180
agatgcagag gttgaggtgg ctgcgggact ggaagtcatc gggcagaggt ctcacagcag 240
ccaaggaacc tggggcccgc tcctcccccc tccaggccat gaggattctg cagttaatcc 300
tgcttgctct ggcaacaggg cttgtagggg gagagaccag gatcatcaag gggttcgagt 360
gcaagcctca ctcccagccc tggcaggcag ccctgttcga gaagacgcgg ctactctgtg 420
gggcgacgct catcgccccc agatggctcc tgacagcagc ccactgcctc aagccgtggc 480
cgctacatag ttcacctggg gcagcacaac ctccagaagg aggagggctg tgagcagacc 540
cggacagcca ctgagtcctt cccccacccc ggcttcaaca acagcctccc caacaaagac 600
caccgcaatg acatcatgct ggtgaagatg gcatcgccag tctccatcac ctgggctgtg 660
cgacccctca ccctctcctc acgctgtgtc actgctggca ccagctgcct catttccggc 720
tggggcagca cgtccagccc ccagttacgc ctgcctcaca ccttgcgatg cgccaacatc 780
accatcattg agcaccagaa gtgtgagaac gcctaccccg gcaacatcac agacaccatg 840
gtgtgtgcca gcgtgcagga agggggcaag gactcctgcc agggtgactc cgggggccct 900
ctggtctgta accagtctct tcaaggcatt atctcctggg gccaggatcc gtgtgcgatc 960
acccgaaagc ctggtgtcta cacgaaagtc tgcaaatatg tggactggat ccaggagacg 1020
atgaagaaca attagactgg acccacccac cacagcccat caccctccat ttccacttgg 1080
tgtttggttc ctgttcactc tgttaataag aaaccctaag ccaagaccct ctacgaacat 1140
tctttgggcc tcctggacta caggagatgc tgtcacttaa taatcaacct ggggttcgaa 1200
atcagtgaga cctggattca aattctgcct tgaaatattg tgactctggg aatgacaaca 1260
cctggtttgt tctctgttgt atccccagcc ccaaagacag ctcctgccat atatcaagtt 1320
tcaataaata tttct 1335

<210> 7
<211> 1079
<212> DNA
<213> Homo sapiens

<220>
<221> unsure
<222> (268)

<220>
<221> unsure
<222> (688)

<220>
<221> unsure
<222> (700)

<400> 7

tttttgaaga atgccctgca aggcatcaac tggaatgtgt ttattaccaa acaagacaga 60
agagaaccag ggcctgactt ggcagtggcc ccaggctgca tgggctcagg taggctcaga 120
ccggccccag gagtgggaga gcccagagaa gagggaaaaa gagtagtggc caggaggggt 180
ctggctggga catgccactc tgggccatca gcttctggat ccactcaaag tggtggctga 240
tattggtgta gacaccgggc cgattggncc gaccacagcc cactccccag ctcacgactc 300
caatctgata ccacagtcca ttcttgttac aggccaaggg tccacctgag tcaccgaagc 360
aggcatcctt cccgccttgg gcattgccag cacaaaccat gtctccaaag atgtccttgc 420
ggaaactgta cttgaggaag aggtggttgc acatagagtt gtttatgatg gcgacctgaa 480
cttcctggag ggtgtgggga gatggcagtg cctcatcctc tttgatgtac ccccagccag 540
tcacccagca gtctgtccgg ttctcaaact caaatgtgga ggcctggaga cagatgggct 600
ggatgtgttt agtgtaggtg acaggtgcag acagcttcac caaggcaatg tcatagggtg 660
aattccccag ttagcgaggg ctcagatnga tattcgatan gaagtaacgg gtgtagtagg 720
cctgcaggct ccagaaggat ggcatggaag tcagctggcc aaactggacc atccacccgg 780
agggatcact aaggtcacta taggtttcaa agcagtgcgc cgccgtgagt gcccagcggt 840
ggctgagcag gctcactccg catacgtggg aatcccacag gcgcaggctc ccctgccacg 900
gccaacgccc gagttcggcg tcctctccac ccacgatgcg cgacgtgatg acccgtcggc 960
cgcatggtcc tgataagggc gccgcctcct gcgactccgg cttcctgagt ccagcccgag 1020
ccagcagcag cgccagcagc agcgccccgc gcgcgcccat ggcctcctct cccgcggtg 1079

## Description

### FIELD OF THE INVENTION

This invention relates, in part, to newly developed assays for detecting, diagnosing, monitoring, staging, and prognosticating cancers, particularly prostate cancer.

### BACKGROUND OF THE INVENTION

Cancer of the prostate is the most prevalent malignancy in adult males, excluding skin cancer, and is an increasingly prevalent health problem in the United States. In 1996, it was estimated that in the United States, 41,400 deaths would result from this disease, indicating that prostate cancer is second only to lung cancer as the most common cause of death in the same population. If diagnosed and treated early, when the cancer is still confined to the prostate, the chance of cure is significantly higher.

Treatment decisions for an individual are linked to the stage of prostate cancer present in that individual. A common classification of the spread of prostate cancer was developed by the American Urological Association (AUA). The AUA classification divides prostate tumors into four stages, A to D. Stage A, microscopic cancer within prostate, is further subdivided into stages A1 and A2. Sub-stage A1 is a well-differentiated cancer confined to one site within the prostate. Treatment is generally observation, radical prostatectomy, or radiation. Sub-stage A2 is a moderately to poorly differentiated cancer at multiple sites within the prostate. Treatment is radical prostatectomy or radiation. Stage B, palpable lump within the prostate, is further subdivided into stages B1 and B2. In sub-stage B1, the cancer forms a small nodule in one lobe of the prostate. In sub-stage B2, the cancer forms large or multiple nodules, or occurs in both lobes of the prostate. Treatment for both sub-stages B1 and B2 is either radical prostatectomy or radiation. Stage C is a large cancer mass involving most or all of the prostate and is further subdivided into two stages. In sub-stage C1, the cancer forms a continuous mass that may have extended beyond the prostate. In sub-stage C2, the cancer forms a continuous mass that invades the surrounding tissue. Treatment for both these sub-stages is radiation with or without drugs. The fourth stage is metastatic cancer and is also subdivided into two stages. In sub-stage D1, the cancer appears in the lymph nodes of the pelvis. In sub-stage D2, the cancer involves tissues beyond lymph nodes. Treatment for both these sub-stages is systemic drugs to address the cancer as well as pain.

However, current prostate cancer staging methods are limited. As many as 50% of prostate cancers initially staged as A2, B, or C are actually stage D, metastatic. Discovery of metastasis is significant because patients with metastatic cancers have a poorer prognosis and require significantly different therapy than those with localized cancers. The five year survival rates for patients with localized and metastatic prostate cancers are 93% and 29%, respectively.

Accordingly, there is a great need for increasingly sensitive methods for the staging of a cancer in a human to determine whether or not such cancer has metastasized and for monitoring the progress of a cancer in a human.

In the present invention, methods are provided for detecting, diagnosing, monitoring, staging and prognosticating cancers, particularly prostate cancer via seven (7) Prostate Specific Genes (PSG). The seven PSGs refer, among other things, to native proteins expressed by the genes comprising the polynucleotide sequences of any of SEQ ID NO: 1, 2, 3, 4, 5, 6 or 7. In the alternative, what is meant by the seven PSGs as used herein, means the native mRNAs encoded by the genes comprising any of the polynucleotide sequences of SEQ ID NO: 1, 2, 3, 4, 5, 6 or 7 or levels of the genes comprising any of the polynucleotide sequences of SEQ ID NO: 1, 2, 3, 4, 5, 6 or 7.

Other objects, features, advantages and aspects of the present invention will become apparent to those of skill in the art from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### SUMMARY OF THE INVENTION

Toward these ends, and others, it is an object of the present invention to provide a method for diagnosing the presence of prostate cancer in a patient which comprises measuring levels of PSG in a sample of cells, tissue or bodily fluid from the patient and comparing the measured levels of PSG with levels of PSG in preferably the same cells, tissue, or bodily fluid type of a control, wherein an increase in the measured PSG levels in the patient versus levels of PSG in the control is associated with prostate cancer.

Another object of the present invention is to provide a method of diagnosing metastatic prostate cancer in a patient which comprises measuring PSG levels in a sample of cells, tissue, or bodily fluid from the patient and comparing the measured PSG levels with levels of PSG in preferably the same cells, tissue, or bodily fluid type of a control, wherein an increase in measured PSG levels in the patient versus levels of PSG in the control is associated with a cancer which has metastasized.

Another object of the present invention is to provide a method of staging prostate cancer in a patient which comprises identifying a patient having prostate cancer, measuring levels of PSG in a sample of cells, tissues, or bodily fluid obtained from the patient, and comparing the measured PSG levels with levels of PSG in preferably the same cells, tissue or bodily fluid type of a control. An increase in measured PSG levels in the patient versus PSG levels in the control can be associated with a cancer which is progressing while a decrease or equivalent level of PSG measured in the patient versus the control can be associated with a cancer which is regressing or in remission.

Another object of the present invention is to provide a method of monitoring prostate cancer in a patient for the onset of metastasis. The method comprises identifying a patient having prostate cancer that is not known to have metastasized, periodically measuring levels of PSG in a sample of cells, tissues, or bodily fluid obtained from the patient, and comparing the measured PSG levels with levels of PSG in preferably the same cells, tissue, or bodily fluid type of a control, wherein an increase in measured PSG levels versus control PSG levels is associated with a cancer which has metastasized.

Yet another object of the present invention is to provide a method of monitoring the change in stage of prostate cancer in a patient which comprises identifying a patient having prostate cancer, periodically measuring levels of PSG in a sample of cells, tissue, or bodily fluid obtained from the patient, and comparing the measured PSG levels with levels of PSG in preferably the same cells, tissues, or bodily fluid type of a control wherein an increase in measured PSG levels versus the control PSG levels is associated with a cancer which is progressing and a decrease in the measured PSG levels versus the control PSG levels is associated with a cancer which is regressing or in remission.

Other objects, features, advantages and aspects of the present invention will become apparent to those of skill in the art from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### DESCRIPTION OF THE INVENTION

The present invention relates to diagnostic assays and methods, both quantitative and qualitative for detecting, diagnosing, monitoring, staging, and prognosticating cancers by comparing levels of PSG measured in a patient with levels of PSG in a control. What is meant by "levels of PSG" as used herein, means levels of the native protein expressed by the gene comprising the polynucleotide sequence of any of SEQ ID NO: 1, 2, 3, 4, 5, 6 or 7. In the alternative, what is meant by "levels of PSG" as used herein, is levels of the native mRNA encoded by the gene comprising any of the polynucleotide sequence of SEQ ID NO: 1, 2, 3, 4, 5, 6 or 7 or levels of the gene comprising any of the polynucleotide sequence of SEQ ID NO: 1, 2, 3, 4, 5, 6 or 7. Such levels are preferably measured in at least one of cells, tissues and/or bodily fluids, and includes determination of both normal and abnormal levels of PSGs. Thus, for instance, a diagnostic assay in accordance with the invention for diagnosing overexpression of PSG protein compared to control bodily fluids, cells, or tissue samples may be used to diagnose the presence of cancers, including prostate cancer. Any of the seven PSGs may be measured alone in the methods of the invention, all together or in various combinations of the seven PSGs.

By "control" it is meant a human patient without cancer and/or non cancerous samples from the patient, also referred to herein as a normal human control; in the methods for diagnosing or monitoring for metastasis, control may also include samples from a human patient that is determined by reliable methods to have prostate cancer which has not metastasized.

All the methods of the present invention may optionally include measuring the levels of other cancer markers as well as PSG. Other cancer markers, in addition to PSG, useful in the present invention will depend on the cancer being tested and are known to those of skill in the art. For example, simultaneous testing for increases in PSA as well as increases in PSG are also within the scope of the present invention and believed to provide a higher level of assurance that such cancer being tested is metastatic or the onset of metastasis has occurred.

### Diagnostic Assays

The present invention provides methods for diagnosing the presence of prostate cancer by analyzing for changes in levels of PSG in cells, tissues or bodily fluids compared with levels of PSG in cells, tissues or bodily fluids of preferably the same type from a normal human control, wherein an increase in levels of PSG in the patient versus the normal human control is associated with the presence of prostate cancer. Without limiting the instant invention, typically, for a quantitative diagnostic assay a positive result indicating the patient being tested has cancer is one in which cells, tissues, or bodily fluid levels of the cancer marker, such as PSG, are at least two times higher, and most preferably are at least five times higher, than in preferably the same cells, tissues, or bodily fluid of a normal human control.

The present invention also provides a method of diagnosing metastatic prostate cancer in a patient having prostate cancer which has not yet metastasized for the onset of metastasis. In the method of the present invention, a human cancer patient suspected of having prostate cancer which may have metastasized (but which was not previously known to have metastasized) is identified. This is accomplished by a variety of means known to those of skill in the art. For example, in the case of prostate cancer, patients are typically diagnosed with prostate cancer following traditional detection methods.

In the present invention, determining the presence of PSG in cells, tissues, or bodily fluid, is particularly useful for discriminating between prostate cancer which has not metastasized and prostate cancer which has metastasized.

Existing techniques have difficulty discriminating between prostate cancer which has metastasized and prostate cancer which has not metastasized and proper treatment selection is often dependent upon such knowledge.

In the present invention, the cancer marker levels measured in such cells, tissue, or bodily fluid are PSGs, and are compared with levels of PSG in preferably the same cells, tissue, or bodily fluid type of a normal human control. That is, if the cancer marker being observed is just PSG in serum, this level is preferably compared with the level of PSG in serum of a normal human patient. An increase in the PSG in the patient versus the normal human control is associated with prostate cancer which has metastasized.

Without limiting the instant invention, typically, for a quantitative diagnostic assay a positive result indicating the cancer in the patient being tested or monitored has metastasized is one in which cells, tissues, or bodily fluid levels of the cancer marker, such as PSG, are at least two times higher, and most preferable are at least five times higher, than in preferably the same cells, tissues, or bodily fluid of a normal patient.

### Staging

The invention also provides a method of staging prostate cancer in a human patient.

The method comprises identifying a human patient having such cancer and analyzing a sample of cells, tissues, or bodily fluid from such patient for PSG. Then, the method compares PSG levels in such cells, tissues, or bodily fluid with levels of PSG in preferably the same cells, tissues, or bodily fluid type of a normal human control sample, wherein an increase in PSG levels in the patient versus the normal human control is associated with a cancer which is progressing and a decrease in the levels of PSG is associated with a cancer which is regressing or in remission.

### Monitoring

Further provided is a method of monitoring prostate cancer in a human having such cancer for the onset of metastasis. The method comprises identifying a human patient having such cancer that is not known to have metastasized; periodically analyzing a sample of cells, tissues, or bodily fluid from such patient for PSG; and comparing the PSG levels in such cells, tissue, or bodily fluid with levels of PSG in preferably the same cells, tissues, or bodily fluid type of a normal human control sample, wherein an increase in PSG levels in the patient versus the normal human control is associated with a cancer which has metastasized.

Further provided by this invention is a method of monitoring the change in stage of prostate cancer in a human having such cancer. The method comprises identifying a human patient having such cancer; periodically analyzing a sample of cells, tissue, or bodily fluid from such patient for PSG; comparing the PSG levels in such cells, tissue, or bodily fluid with levels of PSG in preferably the same patient.

Monitoring such patient for onset of metastasis is periodic and preferably done on a quarterly basis. However, this may be more or less frequent depending on the cancer, the particular patient, and the stage of the cancer.

### Assay Techniques

Assay techniques that can be used to determine levels of gene expression, such as PSG of the present invention, in a sample derived from a host are well-known to those of skill in the art. Such assay methods include radioimmunoassays, reverse transcriptase PCR (RT-PCR) assays, immunohistochemistry assays, *in situ* hybridization assays, competitive-binding assays, Western Blot analyses and ELISA assays. Among these, ELISAs are frequently preferred to diagnose a gene's expressed protein in biological fluids. An ELISA assay initially comprises preparing an antibody, if not readily available from a commercial source, specific to PSG, preferably a monoclonal antibody. In addition a reporter antibody generally is prepared which binds specifically to PSG. The reporter antibody is attached to a detectable reagent such as radioactive, fluorescent or enzymatic reagent, for example horseradish peroxidase enzyme or alkaline phosphatase.

To carry out the ELISA, antibody specific to PSG is incubated on a solid support, e.g., a polystyrene dish, that binds the antibody. Any free protein binding sites on the dish are then covered by incubating with a non-specific protein such as bovine serum albumin. Next, the sample to be analyzed is incubated in the dish, during which time PSG binds to the specific antibody attached to the polystyrene dish. Unbound sample is washed out with buffer. A reporter antibody specifically directed to PSG and linked to horseradish peroxidase is placed in the dish resulting in binding of the reporter antibody to any monoclonal antibody bound to PSG. Unattached reporter antibody is then washed out. Reagents for peroxidase activity, including a colorimetric substrate are then added to the dish. Immobilized peroxidase, linked to PSG antibodies, produces a colored reaction product. The amount of color developed in a given time period is proportional to the amount of PSG protein present in the sample. Quantitative results typically are obtained by reference to a standard curve.

A competition assay may be employed wherein antibodies specific to PSG attached to a solid support and labeled PSG and a sample derived from the host are passed over the solid support and the amount of label detected attached to the solid support can be correlated to a quantity of PSG in the sample.

Nucleic acid methods may be used to detect PSG mRNA as a marker for prostate cancer. Polymerase chain reaction (PCR) and other nucleic acid methods, such as ligase chain reaction (LCR) and nucleic acid sequence based amplification (NASABA), can be used to detect malignant cells for diagnosis and monitoring of various malignancies. For example, reverse-transcriptase PCR (RT-PCR) is a powerful technique which can be used to detect the presence of a specific mRNA population in a complex mixture of thousands of other mRNA species. In RT-PCR, an mRNA species is first reverse transcribed to complementary DNA (cDNA) with use of the enzyme reverse transcriptase; the cDNA is then amplified as in a standard PCR reaction. RT-PCR can thus reveal by amplification the presence of a single species of mRNA. Accordingly, if the mRNA is highly specific for the cell that produces it, RT-PCR can be used to identify the presence of a specific type of cell.

Hybridization to clones or oligonucleotides arrayed on a solid support (i.e., gridding) can be used to both detect the expression of and quantitate the level of expression of that gene. In this approach, a cDNA encoding the PSG gene is fixed to a substrate. The substrate may be of any suitable type including but not limited to glass, nitrocellulose, nylon or plastic. At least a portion of the DNA encoding the PSG gene is attached to the substrate and then incubated with the analyte, which may be RNA or a complementary DNA (cDNA) copy of the RNA, isolated from the tissue of interest.

Hybridization between the substrate bound DNA and the analyte can be detected and quantitated by several means including but not limited to radioactive labeling or fluorescence labeling of the analyte or a secondary molecule designed to detect the hybrid. Quantitation of the level of gene expression can be done by comparison of the intensity of the signal from the analyte compared with that determined from known standards. The standards can be obtained by *in vitro* transcription of the target gene, quantitating the yield, and then using that material to generate a standard curve.

The above tests can be carried out on samples derived from a variety of patients' cells, bodily fluids and/or tissue extracts (homogenates or solubilized tissue) such as from tissue biopsy and autopsy material. Bodily fluids useful in the present invention include blood, urine, saliva, or any other bodily secretion or derivative thereof. Blood can include whole blood, plasma, serum, or any derivative of blood.

### EXAMPLES

The present invention is further described by the following examples. These examples are provided solely to illustrate the invention by reference to specific embodiments. These exemplifications, while illustrating certain specific aspects of the invention, do not portray the limitations or circumscribe the scope of the disclosed invention.

### EXAMPLE 1: PSGs

Searches were carried out and PSGs identified using the following Search Tools as part of the LIFESEQ® database available from Incyte Pharmaceuticals, Palo Alto, CA:
1. Library Comparison (compares one library to one other library) allows the identification of clones expressed in tumor and absent or expressed at a lower level in normal tissue.
2. Subsetting is similar to library comparison but allows the identification of clones expressed in a pool of libraries and absent or expressed at a lower level in a second pool of libraries.
3. Transcript Imaging lists all of the clones in a single library or a pool of libraries based on abundance. Individual clones can then be examined using Electronic Northerns to determine the tissue sources of their component ESTs .
4. Protein Function: Incyte has identified subsets of ESTs with a potential protein function based on homologies to known proteins. Some examples in this database include Transcription Factors and Proteases. Some leads were identified by searching in this database for clones whose component ESTs showed disease specificity.

Electronic subtractions, transcript imaging and protein function searches were used to identify clones, whose component ESTs were exclusively or more frequently found in libraries from specific tumors. Individual candidate clones were examined in detail by checking where each EST originated.

**Table 1:**

| **SEQ ID NO:** | **Clone ID #** | **Gene ID #** | |
|---|---|---|---|
| 1 | 1550426 | 244673 | Protein Function (Transcription Factors) |
| 2 | 1255804 | 14878 | Subsetting |
| 3 | 1808432 | 255819 | Subsetting |
| 4 | 3930803 | none | Subsetting |
| 5 | 645804 | 235032 | Subsetting |
| 6 | 1862352 | 221558 | Subsetting |
| 7 | 1450626 | 236019 | Subsetting |

### EXAMPLE 2: Measurement of SEQ ID NO:1; Clone ID # 1550426; Gene ID #244673 (pro101)

The example is carried out using standard techniques, which are well known and routine to those of skill in the art, except where otherwise described in detail. Routine molecular biology techniques of the following example are carried out as described in standard laboratory manuals, such as Sambrook et al., MOLECULAR CLONING: A LABORATORY MANUAL, 2nd Ed.; Cold Spring Harbor Laboratory Press, Cold Spring Harbor, N.Y. (1989).

### Relative Quantitation of Gene Expression

Real-time quantitative PCR with fluorescent Taqman probes is a quantitative detection system utilizing the 5'-3' nuclease activity of Taq DNA polymerase. The method uses an internal fluorescent oligonucleotide probe (Taqman) labeled with a 5' reporter dye and a downstream, 3' quencher dye. During PCR, the 5'-3' nuclease activity of Taq DNA polymerase releases the reporter, whose fluorescence can then be detected by the laser detector of the Model 7700 Sequence Detection System (PE Applied Biosystems, Foster City, CA, USA).

Amplification of an endogenous control is used to standardize the amount of sample RNA added to the reaction and normalize for Reverse Transcriptase (RT) efficiency. Either cyclophilin, glyceraldehyde-3-phosphate dehydrogenase (GAPDH) or 18S ribosomal RNA (rRNA) is used as this endogenous control. To calculate relative quantitation between all the samples studied, the target RNA levels for one sample are used as the basis for comparative results (calibrator). Quantitation relative to the "calibrator" is obtained using the standard curve method or the comparative method (User Bulletin #2: ABI PRISM 7700 Sequence Detection System).

To evaluate the tissue distribution, and the level of pro101 (SEQ ID NO:1) in normal and tumor tissue, total RNA was extracted from tumor and matched normal adjacent tissues and from unmatched tumor and normal tissues. Subsequently, first strand cDNA was prepared with reverse transcriptase and the polymerase chain reaction carried out using primers and Taqman probe specific to pro101 (SEQ ID NO:1). The results were obtained using the ABI PRISM 7700 Sequence Detector. The absolute numbers are relative levels of expression of pro101 (SEQ ID NO:1) compared to the calibrator.

The absolute numbers are depicted in the following Table 2 as relative levels of expression in 12 normal tissues of pro101 (SEQ ID NO:1) compared to kidney (calibrator). These RNA samples were generated by pooling samples from a particular tissue from different individuals.

**Table 2: Relative levels of pro101 Expression in Pooled Samples**

| **Tissue** | **NORMAL** |
|---|---|
| Brain | 1.2 |
| Heart | 2 |
| Kidney | 1 |
| Liver | 7.2 |
| Lung | 48.2 |
| Mammary | 2.5 |
| Prostate | 1418.4 |
| Spleen | 1.6 |
| Small | 1.9 |
| Testis | 57.3 |
| Thymus | 1.3 |
| Uterus | 7.6 |

The relative levels of expression in Table 2 show that for the PSG pro101 (SEQ ID NO:1) mRNA expression is more than 20 fold higher in the pool of normal prostate compared with the other 11 normal tissue pools analyzed. These results demonstrate that mRNA expression of the PSG is highly specific for prostate.

The tissues shown in Table 2 correspond to pools of samples from different individuals. The tissues shown in the following Table 3 were obtained from individuals and are not pooled. Hence the values for mRNA expression levels shown in Table 2 cannot be directly compared to the values shown in Table 3.

The absolute numbers in Table 3 are relative levels of expression of pro101 (SEQ ID NO:1) compared to kidney (calibrator), in 60 pairs of matching samples. Each matching pair contains the cancer sample for a particular tissue and the normal adjacent sample for that same tissue from the same individual. The results from 3 unmatched ovary tumor, 3 unmatched normal ovary, 1 unmatched mammary tumor and 1 unmatched normal mammary gland are also shown.

**Table 3: Relative Levels of pro101 Expression in Individual Samples**

| **TISSUE** | **CANCER** | **MATCHING** | **UNMATCHED** |
|---|---|---|---|
| Prostate 1 | 103.9 | 0 | |
| Prostate 2 | 2219 | 84.2 | |
| Prostate 3 | 5048.2 | 3623.6 | |
| Prostate 4 | 11052.3 | 2029.4 | |
| Prostate 5 | 229.1 | 41.1 | |
| Prostate 6 | 57.9 | 25.3 | |
| Prostate 7 | 58.5 | 57.069 | |
| Prostate 8 | 1074.6 | 610.8 | |
| Prostate 9 | 32.7 | 79.3 | |
| Prostate 10 | 15.8 | 2.09 | |
| Prostate 11 | 436.4 | 438 | |
| Prostate 12 | 49.5 | 59.3 | |
| Prostate 13 | 128 | 56 | |
| Bladder 1 | 0 | 0 | |
| Bladder 2 | 0 | 0 | |
| Bladder 3 | 0.7 | 0 | |
| Colon 1 | 0 | 0 | |
| Colon 2 | 0 | 0 | |
| Colon 3 | 0 | 0 | |
| Colon 4 | 3.3 | 1.9 | |
| Colon 5 | 0.1 | 0.8 | |
| Colon 6 | 0 | 0 | |
| Lung 1 | 0 | 0 | |
| Lung 2 | 0.5 | 1.6 | |
| Lung 3 | 1.4 | 2.1 | |
| Lung 4 | 0 | 0 | |
| Lung 5 | 0 | 0 | |
| Kidney 1 | 0 | 0 | |
| Kidney 2 | 0 | 0 | |
| Kidney 3 | 0 | 0 | |
| Kidney 4 | 0 | 0 | |
| Liver 1 | 1.5 | 5.7 | |
| Liver 2 | 26.9 | 7.9 | |
| Liver 3 | 0 | 0 | |
| Pancreas 1 | 0.9 | 0.9 | |
| Pancreas 2 | 3 | 0 | |
| Pancreas 3 | 0 | 0 | |
| Pancreas 4 | 0 | 0 | |
| Pancreas 5 | 0 | 0 | |
| Stomach 1 | 0 | 0 | |
| Stomach 2 | 0 | 0 | |
| Stomach 3 | 0 | 0 | |
| Stomach 4 | 0 | 0 | |
| Stomach 5 | 0 | 0 | |
| Sm Int 1 | 0 | 0 | |
| Sm Int 2 | 0 | 0 | |
| Testis 1 | 0 | 0 | |
| Mammary 1 | 4 | 0 | |
| Mammary 2 | 5.6 | 0 | |
| Mammary 3 | 0.5 | 0 | |
| Mammary 4 | 0.4 | 0 | |
| Mammary 5 | 0.5 | | |
| Mammary 6 | | | 0 |
| Endo 1 | 1.6 | 7.6 | |
| Endo 2 | 0 | 0 | |
| Endo 3 | 0 | 0 | |
| Endo 4 | 0.3 | 0.2 | |
| Endo 5 | 5.8 | 5 | |
| Uterus 1 | 0 | 0 | |
| Uterus 2 | 0 | 0 | |
| Uterus 3 | 0 | 0 | |
| Uterus 4 | 2.2 | 2.6 | |
| Ovary 1 | 1.4 | | |
| Ovary 2 | | | 11.6 |
| Ovary 3 | 1.5 | | |
| Ovary 4 | | | 22.9 |
| Ovary 5 | 0 | | |
| Ovary 6 | | | 1.8 |

Among 128 samples in Table 3 representing 14 different tissues, the higher levels of expression are consistently in prostate tissues. These results confirm the tissue specificity results obtained with normal samples shown in Table 2. Table 2 and Table 3 represent a combined total of 140 samples in 18 human tissue types. Sixty-eight samples representing 13 different tissue types excluding prostate had no detected pro101 mRNA (Table 3). In 4 tissues (stomach small intestine kidney and testis) no pro101 (SEQ ID NO:1) mRNA was detected for any sample tested from individuals (Table 3). Expression of this PSG was detected in testis in the pooled normal sample (Table 3). The median expression in prostate cancer samples in Table 3 is 166.5 units. Excluding Ovary 4 (Normal), only 1 sample in Table 3, Liver 2 (Cancer), is greater than 10% of this value.

Comparisons of the level of mRNA expression in prostate tumor samples and the normal adjacent tissue from the same individuals are also shown in Table 3. The PSG pro101 (SEQ ID NO:1) is expressed at higher levels in 9 of 13 (69%) prostate cancer tissues (Prostate 1, 2, 3, 4, 5, 6, 8, 10 and 13) compared with the corresponding normal adjacent tissue. The level of expression of this PSG is lower in prostate tumor compared to normal adjacent tissue in two samples (Prostate 9 and 12). Equivalent levels of expression were detected in two matched samples (Prostate 7 and 11). Previous mRNA expression analysis for genes coding for the diagnostic markers PSA and PLA2 showed higher expression of the mRNA in 40% to 80% of the tumor samples compared to matching normal adjacent tissue. Higher expression in the tumor sample compared to the corresponding normal adjacent tissue is observed for Bladder 3, Colon 4, Liver 2, Pancreas 2, Endometrium 5 and. Mammary 1, 2 and 3. Higher expression in the normal adjacent samples is observed for Colon 5, Lung 2, Lung 3, Liver 1, Endometrium 1 and Uterus 4. However, the levels detected are in most cases comparable amongst the different tissues and low compared to levels found in most prostate tissues.

The high level of tissue specificity, plus the mRNA overexpression in 9 of 13 of the prostate tumor samples tested compared to the normal adjacent tissues are believed to make the PSG, pro101 (SEQ ID NO:1) a good diagnostic marker for detection of prostate cancer using mRNA.

## Claims

1. A diagnostic method indicative of the presence of prostate cancer in a patient, the method comprising:
(a) measuring levels of prostate specific genes (PSG) in a sample of cells, tissue or bodily fluid obtained from the patient; and
(b) comparing the measured levels of PSG with levels of PSG in a sample of cells, tissue or bodily fluid obtained from a control, wherein an increase in measured levels of PSG in the patient versus the PSG levels in the control is associated with the presence of prostate cancer.

2. A diagnostic method indicative of the presence of metastatic prostate cancer in a patient, the method comprising:
(a) measuring levels of PSG in a sample of cells, tissue, or bodily fluid obtained from the patient; and
(b) comparing the measured levels of PSG with levels of PSG in a sample of cells, tissue, or bodily fluid obtained from a control, wherein an increase in measured PSG levels in the patient versus the PSG levels in the control is associated with a cancer which has metastasized.

3. A method of staging prostate cancer in a patient, the method comprising:
(a) measuring levels of PSG in a sample of cells, tissue, or bodily fluid obtained from the patient; and.
(b) comparing the measured levels of PSG with levels of PSG in a sample of cells, tissue, or bodily fluid obtained from a control, wherein an increase in the measured levels of PSG versus the levels of PSG in the control is associated with a cancer which is progressing and a decrease in the measured levels of PSG versus the levels of PSG in the control is associated with a cancer which is regressing or in remission.

4. A method of monitoring prostate cancer in a patient for the onset of metastasis, the method comprising:
(a) periodically measuring PSG levels in samples of cells, tissue, or bodily fluid obtained from the patient having prostate cancer that is not known to have metastasized; and
(b) comparing the periodically measured levels of PSG with levels of PSG in cells, tissue, or bodily fluid obtained from a control, wherein an increase in any one of the periodically measured levels of PSG in the patient versus the levels of PSG in the control is associated with a cancer which has metastasized.

5. A method of monitoring changes in a stage of prostate cancer in a patient, the method comprising:
(a) periodically measuring levels of PSG in samples of cells, tissue, or bodily fluid obtained from the patient; and
(b) comparing the measured levels of PSG with levels of PSG in a sample of the same cells, tissue, or bodily fluid of a control, wherein an increase in any one of the periodically measured levels of PSG versus levels of PSG in the control is associated with a cancer which is progressing in stage and a decrease in any one of the periodically measured levels of PSG versus the levels of PSG in the control is associated with a cancer which is regressing in stage or in remission.

6. A method according to any preceding claim wherein the PSG comprises SEQ ID NO:7.

7. A method according to any of claims 1 to 5 wherein the PSG comprises SEQ ID NO:1.
